# EUROPÄISCHE PATENTSCHRIFT

(11) **EP 3 452 135 B1**
(45) Veröffentlichungstag und Bekanntmachung des Hinweises auf die Patenterteilung: **23.12.2020**
(21) Anmeldenummer: 17721389.9
(22) Anmeldetag: 04.05.2017
(51) Int. Cl.: A61M 1/10, F04B 43/12, F04B 43/00, F04B 49/06

(54) **MEDIZINISCHE BEHANDLUNGSVORRICHTUNG UND SCHLAUCHSET FÜR EINE MEDIZINISCHE BEHANDLUNGSVORRICHTUNG**
MEDICAL TREATMENT DEVICE AND HOSE SET FOR A MEDICAL TREATMENT DEVICE
DISPOSITIF DE TRAITEMENT MÉDICAL ET ENSEMBLE DE TUYAUX SOUPLES POUR UN DISPOSITIF DE TRAITEMENT MÉDICAL

(30) Priorität: 06.05.2016 DE 102016005467
(43) Veröffentlichungstag der Anmeldung: 13.03.2019
(73) Patentinhaber: Fresenius Medical Care Deutschland GmbH, 61352 Bad Homburg v.d.H (DE)
(72) Erfinder: HEIDE, Alexander, 65817 Eppstein (DE); PETERS, Arne, 61352 Bad Homburg (DE); NIKOLIC, Dejan, 65824 Bad Soden (DE)
(74) Vertreter: Oppermann, Frank
(86) Internationale Anmeldenummer: PCT/EP2017/060721
(87) Internationale Veröffentlichungsnummer: WO 2017/191292

(56) Entgegenhaltungen:
- EP-A2- 0 542 140
- EP-A2- 0 745 400
- WO-A1-2005/111424
- WO-A1-2012/110313
- US-A- 4 411 651

## Beschreibung

Die Erfindung betrifft eine medizinische Behandlungsvorrichtung, insbesondere eine extrakorporale Blutbehandlungsvorrichtung, mit einem Schlauchset und einer peristaltischen Schlauchpumpe zum Fördern von Flüssigkeit sowie einer Überwachungsvorrichtung zur Überwachung der Okklusion der Verdrängerkörper der peristaltischen Schlauchpumpe. Darüber hinaus betrifft die Erfindung ein Schlauchset für eine medizinische Behandlungsvorrichtung, insbesondere eine extrakorporale Blutbehandlungsvorrichtung.

Bei einer extrakorporalen Blutbehandlung durchströmt das zu behandelnde Blut in einem extrakorporalen Blutkreislauf die Blutkammer eines durch eine semipermeable Membran in die Blutkammer und eine Dialysierflüssigkeitskammer unterteilten Dialysators, während in einem Dialysierflüssigkeitssystem Dialysierflüssigkeit die Dialsysierflüssigkeitskammer des Dialysators durchströmt. Der extrakorporale Blutkreislauf weist eine arterielle Schlauchleitung auf, die zu der Blutkammer führt, und eine venöse Schlauchleitung auf, die von der Blutkammer abgeht. Die Schlauchleitungen der extrakorporalen Blutbehandlungsvorrichtung werden im Allgemeinen als zur einmaligen Verwendung bestimmtes Schlauchset (Disposable) bereitgestellt. Die bekannten Blutbehandlungsvorrichtungen verfügen über eine Blutpumpe, die im Allgemeinen stromauf der Blutkammer des Dialysators angeordnet ist, um einen ausreichenden Blutfluss im extrakorporalen Blutkreislauf sicher zu stellen.

An die Blutpumpen werden hohe technische Anforderungen gestellt. Daher kommen nur bestimmte Pumpentypen in Betracht. In der Praxis haben sich Schlauchpumpen bewährt, die das Blut des Patienten durch die arterielle und venöse Schlauchleitung fördern.

Die Schlauchpumpen werden nach ihrer Arbeitsweise auch als peristaltische Pumpen bezeichnet. Ihre Pumpwirkung basiert darauf, dass sich mindestens eine Eng- oder Verschlussstelle (Okklusion) längs der als Pumpenraum dienenden elastischen Schlauchleitung bewegt und dadurch die eingeschlossene Flüssigkeit in Förderrichtung verschiebt. Bei der gebräuchlichsten Bauart der Schlauchpumpen wird der elastische Schlauch an den bewegten Engstellen vollständig verschlossen. Daher werden diese Pumpen auch als okklusive Schlauchpumpen bezeichnet.

Die beweglichen Eng- oder Verschlussstellen, die das Blut im Pumpenschlauch transportieren, können unterschiedlich ausgebildet sein. Es sind Rollenpumpen bekannt, bei denen der Schlauch zwischen einem Stator, der eine gebogene Rollenbahn als Widerlager bildet, und einem drehbar darin gelagerten, mit Rollen besetzten Rotor eingelegt wird, so dass sich die Rollen in Förderrichtung auf dem Schlauch abwälzen und auf den Schlauch eine Anpresskraft ausüben. Daneben sind auch Fingerpumpen bekannt, bei denen die Verschlusskörper durch eine längs des Schlauchs angeordnete Reihe von beweglichen Stempeln (Fingern) gebildet werden.

Einen Überblick über die unterschiedlichen Bauarten der Rollen- und Fingerpumpen wird in Dialysetechnik, 4. Auflage, Gesellschaft für angewandte Medizintechnik m.b.H und Co. KG, Friedrichsdorf, 1988 gegeben.

Bei der Verwendung in medizintechnischen Einrichtungen, insbesondere Blutbehandlungsvorrichtungen, werden an den ordnungsgemäßen Betrieb von peristaltischen Schlauchpumpen hohe Anforderungen gestellt. Schlauchpumpen werden in den bekannten Blutbehandlungsvorrichtungen nicht nur zur Förderung des Bluts, sondern auch zur Förderung anderer Flüssigkeiten, beispielsweise Dialysierflüssigkeit, eingesetzt.

Während des Betriebs der peristaltischen Schlauchpumpe besteht die Gefahr einer Unterbrechung der Flüssigkeitsströmung in der Schlauchleitung stromab der Schlauchpumpe, beispielsweise durch Abknicken der Schlauchleitung. Wenn die Schlauchpumpe bei einer Verstopfung der Schlauchleitung betrieben wird, besteht die Gefahr, dass die Schlauchleitung platzt. Da die Okklusionskörper bekannter Schlauchpumpen federnd gelagert sind, können diese bei Überschreiten eines vorgegebenen Überdrucks in der Schlauchleitung abheben, so dass sich der Druck in der Schlauchleitung durch Rückfluss der Flüssigkeit abbauen kann. Dadurch ist aber die Okklusion der Schlauchpumpe aufgehoben, so dass ein ordnungsgemäßer Betrieb nicht sichergestellt ist. Insbesondere bei Blutpumpen kann ein dauerhafter Betrieb der Pumpe mit abgehobenen Okklusionskörpern zu einer Schädigung des Bluts im Schlauchsegment führen (Hämolyse), was eine zeitnahe Erkennung der Verstopfung wünschenswert macht.

Die Verstopfung einer Schlauchleitung kann beim Betrieb einer peristaltischen Schlauchpumpe allein mit einer Überwachung des Drucks in der Schlauchleitung nicht sicher erkannt werden, da der Druck bei Abheben der Verdrängerkörper über einen bestimmten Grenzwert hinaus nicht weiter ansteigen kann.

Aus der WO 2007/104435 A2 ist ein Verfahren und eine Vorrichtung zum Betreiben einer peristaltischen Schlauchpumpe, insbesondere einer Schlauchpumpe zum Fördern von Flüssigkeiten in extrakorporalen Blutbehandlungsvorrichtungen, bekannt. Zur Überwachung des ordnungsgemäßen Betriebs der Schlauchpumpe wird die Leistungsaufnahme der Pumpe oder eine mit der Leistungsaufnahme korrelierende physikalische Größe überwacht. Die Überwachung des Pumpenstroms beruht darauf, dass der Pumpenstrom einen sich periodisch nicht ändernden Gleichanteil aufweist, dem ein sich periodisch ändernder Wechselteil überlagert ist.

Die US-A-5,629,871 beschreibt ein Verfahren und eine Vorrichtung zur Überwachung der Funktionsfähigkeit einzelner Baugruppen einer Hämodialysevorrichtung. Zu diesen zählen auch die Schlauchpumpen, wobei der Pumpenstrom oder die Versorgungsspannung der Schlauchpumpen überwacht wird, um einen Ausfall der Pumpe feststellen zu können.

Aus der EP 2 918 837 A1 ist eine extrakorporale Blutbehandlungsvorrichtung bekannt, bei der zur Erkennung einer Verstopfung der Schlauchleitung das Abheben der federnd gelagerten Verdrängerkörper mittels eines Kraftaufnehmers überwacht wird.

Die WO 2012/110313 A1 beschreibt eine Vorrichtung zum Sterilisieren eines medizinischen Instruments, die über eine peristaltische Pumpe zum Pumpen einer Sterilisierungsflüssigkeit und ein Becken zur Aufnahme des medizinischen Instruments verfügt. Die peristaltische Pumpe weist eine Aufnahmeeinheit auf, in die eine Schlauchleitung eingelegt ist, deren Enden mit elektrisch leitfähigen Fittings aus Edelstahl versehen sind. An den Fittings befinden sich Anschlusstücke für einen Einlass- und Auslassschlauch. Die elektrisch leitfähigen Fittings stellen eine elektrische Verbindung zu der in der Schlauchleitung strömenden Flüssigkeit her. Darüber hinaus verfügt die Vorrichtung zum Sterilisieren über eine Einrichtung zum Messen des elektrischen Widerstandes zwischen den Fittings und eine Auswerteeinheit.

Aus der EP 0 745 400 A2 ist eine peristaltische Pumpe bekannt, deren Okklusion mit einer Einrichtung überwacht wird, die über Spulen verfügt, die zum Induzieren eines Wechselstroms an den Enden der in die Pumpe eingelegten Schlauchleitung angeordnet sind. Die Überwachungseinrichtung misst den durch die flüssigkeitsgefüllte Schlauchleitung fließenden Strom.

Die WO 2005/111424 A1 beschreibt eine peristaltische Pumpe mit einer Aufnahmeeinheit, die ein bogenförmiges Pumpenbett aufweist. Das in die Aufnahmeeinheit einzulegende Schlauchsegment bildet eine Schlaufe. Die sich kreuzenden Enden der Schlaufe sind mit einem Anschlussstück fixiert. Die peristaltische Pumpe sieht eine Überwachung der Okklusion nicht vor.

Aus der EP 0 542 140 A2 ist ein Schlauchset für eine extrakorporale Blutbehandlungsvorrichtung bekannt. Das Schlauchset umfasst ein rohrförmiges Schlauchstück, an dem Elektroden zum Anschluss elektrischer Leitungen einer Überwachungsvorrichtung vorgesehen sind. Das rohrförmige Schlauchstück besteht aus mehreren miteinander verbundenen Stücken, die jeweils einen Kanal aufweisen. Die als Elektroden ausgebildeten Stücke bestehen aus einem leitfähigen Material.

Der Erfindung liegt die Aufgabe zugrunde, eine Blockade der Flüssigkeitsströmung in einer Schlauchleitung eines Schlauchsets einer medizinischen Behandlungsvorrichtung, insbesondere einer extrakorporalen Blutbehandlungsvorrichtung, sicher zu erkennen. Darüber hinaus liegt der Erfindung die Aufgabe zugrunde, den ordnungsgemäßen Betrieb einer peristaltischen Schlauchpumpe, insbesondere einer peristaltischen Schlauchpumpe einer extrakorporalen Blutbehandlungsvorrichtung, überwachen zu können. Der Erfindung liegt auch die Aufgabe zugrunde, ein Verfahren bereit zu stellen, das die sichere Erkennung einer Blockade der Flüssigkeitsströmung in einer Schlauchleitung eines Schlauchsets einer medizinischen Behandlungsvorrichtung und die Überwachung des ordnungsgemäßen Betriebs einer peristaltischen Schlauchpumpe ermöglicht. Eine weitere Aufgabe der Erfindung ist, ein einfach zu handhabendes Schlauchset für eine medizinische Behandlungsvorrichtung zu schaffen, das in Verbindung mit einer Überwachungsvorrichtung die Überwachung der Blutbehandlung erlaubt.

Die Lösung dieser Aufgaben erfolgt erfindungsgemäß mit den Merkmalen der unabhängigen Patentansprüche 1 und 8. Vorteilhafte Ausführungsformen der Erfindung sind Gegenstand der abhängigen Ansprüche.

Die Erfindung beruht darauf, dass zur Überwachung der Flüssigkeitsströmung in einer Schlauchleitung die Okklusion der Verdrängerkörper der peristaltischen Schlauchpumpe zum Fördern der Flüssigkeit in der Schlauchleitung überwacht wird. Für die Erfindung ist grundsätzlich unerheblich, welche Flüssigkeit mit der Schlauchpumpe gefördert wird. Allerdings setzt die Erfindung eine bestimmte Leitfähigkeit der Flüssigkeit voraus.

Das Grundprinzip der Erfindung liegt darin, eine Blockade der Flüssigkeitsströmung in der Schlauchleitung nicht durch eine Überwachung von Bauteilen der Verdrängerpumpe selbst, sondern durch Überwachung des elektrischen Widerstandes oder einer mit dem elektrischen Widerstand korrelierenden Größe der Flüssigkeit in einem Schlauchsegment der Schlauchleitung zu erkennen. Hierzu wird der elektrische Widerstand oder eine mit dem elektrischen Widerstand korrelierende Größe zwischen einer ersten und einer zweiten Elektrode gemessen, wobei die erste Elektrode stromauf der Okklusionskörper der peristaltischen Pumpe und die zweite Elektrode stromab der Okklusionskörper derart an der Schlauchleitung angeordnet ist, dass ein elektrischer Kontakt zwischen der ersten und zweiten Elektrode und der in der Schlauchleitung strömenden Flüssigkeit hergestellt ist. Folglich wird nicht die Position der Verdrängerkörper, sondern der elektrische Widerstand oder die mit dem elektrischen Widerstand korrelierenden Größe der Flüssigkeit erfasst.

Der Erfindung liegt die Erkenntnis zugrunde, dass die Verdrängerkörper bei ordnungsgemäßen Betrieb der Schlauchpumpe die Schlauchleitung abquetschen, so dass der elektrische Widerstand, der von der in dem Schlauchsegment befindlichen Flüssigkeit abhängig ist, groß ist bzw. die Leitfähigkeit gering ist. Wenn die Verdrängerkörper bei einem Überdruck in der Schlauchleitung infolge einer Blockade der Flüssigkeitsströmung von der Schlauchleitung abheben, nimmt der elektrische Widerstand in dem betreffenden Schlauchleitungsabschnitt ab bzw. nimmt die Leitfähigkeit zu.

Die erfindungsgemäße medizinische Behandlungsvorrichtung verfügt über ein Schlauchset, das eine oder mehrere Schlauchleitungen aufweisen kann, und eine peristaltische Schlauchpumpe zum Fördern von Flüssigkeit, die eine Aufnahmeeinheit mit einem Pumpenbett zum Einlegen eines Schlauchsegments der Schlauchleitung und bewegliche Okklusionskörper zur Beaufschlagung des in das Pumpenbett eingelegten Schlauchsegments aufweist, sowie eine Überwachungsvorrichtung zur Überwachung der Okklusion der Okklusionskörper der Schlauchpumpe. Die Schlauchpumpe kann unterschiedlich ausgebildet sein, solange die Pumpe über Verdrängerkörper verfügt.

Die Überwachungsvorrichtung weist eine Einrichtung zum Messen des elektrischen Widerstandes oder einer mit dem elektrischen Widerstand korrelierenden Größe zwischen einer ersten und einer zweiten Elektrode auf. Eine mit dem elektrischen Widerstand korrelierende Größe kann die Leitfähigkeit oder der Strom oder die Spannung sein (Ohmsches Gesetz). Die erste Elektrode ist stromauf der Okklusionskörper und die zweite Elektrode ist stromab der Okklusionskörper derart an der Schlauchleitung angeordnet, dass ein elektrischer Kontakt zwischen der ersten und zweiten Elektrode und der in der Schlauchleitung strömenden Flüssigkeit herstellbar ist. Darüber hinaus weist die Überwachungsvorrichtung eine den elektrischen Widerstand oder die mit dem elektrischen Widerstand korrelierende Größe erfassende Rechen- und Auswerteinheit auf.

Die Rechen- und Auswerteinheit kann zur Überwachung der Okklusion unterschiedlich konfiguriert sein. Allein entscheidend ist, dass die Rechen- und Auswerteinheit die Okklusion infolge einer Veränderung des elektrischen Widerstands oder einer mit dem elektrischen Widerstand korrelierenden Größe erkennt, beispielsweise dadurch, dass vorgegebene untere und/oder obere Grenzwerte über- bzw. unterschritten werden.

Eine bevorzugte Ausführungsform sieht vor, dass die Rechen- und Auswerteinheit derart konfiguriert ist, dass eine Veränderung des elektrischen Widerstandes erfasst wird, wobei auf eine mangelnde Okklusion der Okklusionskörper geschlossen wird, wenn der elektrische Widerstand einen vorgegebenen Grenzwert unterschreitet, oder eine Veränderung des elektrischen Leitfähigkeit erfasst wird, wobei auf eine mangelnde Okklusion der Okklusionskörper geschlossen wird, wenn die elektrische Leitfähigkeit einen vorgegebenen Grenzwert überschreitet.

Die Vorteile der erfindungsgemäße Überwachung kommen insbesondere bei einer peristaltischen Pumpe zum tragen, deren Aufnahmeeinheit erfindungsgemäß ein bogenförmiges Pumpenbett aufweist. Bei einer derartigen Rollenpumpe bekannter Bauart bildet das in die Pumpe einzulegende Schlauchsegment eine Schlaufe. Die erste Elektrode und die zweite Elektrode sind vorzugsweise in dem Bereich der sich kreuzenden Schlauchabschnitte der Schlaufe angeordnet, an denen die Elektroden stromauf und stromab der Okklusionskörper relativ dicht nebeneinander liegen und ausreichend fixiert werden können.

Die Erfindung sieht vor, dass das Schlauchset ein Anschlussstück bzw. ein Fixierungsstück aufweist, mit dem die sich kreuzenden Abschnitte des eine Schlaufe bildenden Schlauchsegments fixiert sind, wobei die erste und zweite Elektrode Bestandteil des Anschlussstücks sind. Folglich sind die Elektroden Bestandteil des Anschlussstücks, in dem die Elektroden in einem relativ geringen Abstand zusammen mit der Schlauchleitung ausreichend fixiert sind. Dadurch werden der konstruktive Aufbau und die Handhabung des Schlauchsets vereinfacht. Beispielsweise können sämtliche Anschlussleitungen in einem gemeinsamen Kabel aus dem Anschlussstück herausgeführt zu werden. Darüber hinaus kann die elektrische Verbindung auch dadurch hergestellt werden, dass das Anschlussstück in ein Aufnahmestück eingesetzt wird, wobei an dem Anschlussstück und dem Aufnahmestück entsprechende Kontakte vorgesehen sind. Es ist aber grundsätzlich auch möglich, die Elektroden an anderen Stellen der Schlauchleitung stromauf bzw. stromab der Okklusionskörper vorzusehen.

Das Anschlussstück ist vorzugsweise ein Kunststoffteil, in dem ein erster Kanal mit einem Einlass und einem Auslass und ein zweiter Kanal mit einem Einlass und einem Auslass ausgebildet sind, wobei sich der erste und zweite Kanal vorzugsweise kreuzen, und dass die Elektroden in der Wandung des ersten und zweiten Kanals ausgebildet sind. Das Kunststoffteil ist vorzugsweise ein einstückiges Kunststoffteil.

Erfindungsgemäß besteht der Kunststoff des Kunststoffteils aus einer leitfähigen und einer nicht-leitfähigen Komponente, wobei in dem Bereich des ersten und zweiten Kanals, in dem die erste und zweite Elektrode ausgebildet sind, zur Ausbildung der Elektroden der Kunststoff ein leitfähiger Kunststoff ist. Daraus ergibt sich der Vorteil, dass die Elektroden integraler Bestandteil des Anschlussstücks sind, wodurch die Handhabung besonders einfach ist. Das Anschlussstück kann zusammen mit den Elektroden in großen Stückzahlen kostengünstig hergestellt werden.

Leitfähige Polymere gehören zum Stand der Technik. In einen nicht leitfähigen Kunststoff können beispielsweise Mikrostahlfasern, Kohlenstofffasern oder fein verteilte Metallpartikel eingearbeitet werden.

Das Anschlussstück mit den beiden Komponenten kann mit den bekannten ZweiKomponenten-Spritzgußverfahren (2K-Verfahren) in nur einem Arbeitsgang und mit nur einem Werkzeug einfach und kostengünstig hergestellt werden.

Die Integration einer oder mehrerer Elektroden in ein Kunststoffteil eines Schlauchsets mit einer oder mehreren Schlauchleitungen ist von eigenständiger erfinderischer Bedeutung. Die Elektrode kann für unterschiedliche Zwecke verwendet werden.

Das erfindungsgemäße Schlauchset für eine medizinische Behandlungsvorrichtung zeichnet sich dadurch aus, dass die mindestens eine Elektrode Bestandteil eines aus einer leitfähigen und einer nicht-leitfähigen Komponente bestehenden Kunststoffteils ist, in dem mindestens ein Kanal ausgebildet ist, der einen Einlass aufweist, an den ein Schlauchsegment der Schlauchleitung angeschlossen ist, und einen Auslass aufweist, an den ein Schlauchsegment der Schlauchleitung angeschlossen ist, wobei der Kunststoff in einem Bereich des Kunststoffteils, in dem die Elektrode ausgebildet ist, ein leitfähiger Kunststoff ist. Das Kunststoffteil kann an einer beliebigen Stelle der Schlauchleitung vorgesehen sein. An den Ein- bzw. Auslass des Anschlussstücks können die angrenzenden Schlauchleitungsabschnitte angeschlossen werden. Beispielsweise können diese Abschnitte mit dem Kunststoffteil verklebt oder verschweißt werden.

Nachfolgend wird ein Ausführungsbeispiel der Erfindung unter Bezugnahme auf die Zeichnungen im Einzelnen beschrieben.

Es zeigen:
- FIG. 1: in vereinfachter schematischer Darstellung eine erfindungsgemäße medizinische Behandlungsvorrichtung, die über eine Überwachungsvorrichtung zur Überwachung der Okklusion der peristaltischen Schlauchpumpe verfügt,
- FIG. 2: die peristaltische Schlauchpumpe der medizinischen Behandlungsvorrichtung in vereinfachter schematischer Darstellung
- FIG. 3: das in die peristaltische Schlauchpumpe einzulegende Schlauchsegment zusammen mit einem Anschlussstück und den angrenzenden Schlauchleitungsabschnitten in vereinfachter schematischer Darstellung und
- FIG. 4: das Anschlussstück in vergrößerter Darstellung.

Bei dem vorliegenden Ausführungsbeispiel ist die medizinische Behandlungsvorrichtung eine extrakorporale Blutbehandlungsvorrichtung, insbesondere Hämodialysevorrichtung, die über einen Dialysator 1 verfügt, der durch eine semipermeable Membran 2 in eine Blutkammer 3 und eine Dialysierflüssigkeitskammer 4 unterteilt ist. Von einem Patienten führt ein arterielle Blutleitung 5, in der eine Blutpumpe 6 angeordnet ist, zu einem Einlass der Blutkammer 3, während von einem Auslass der Blutkammer eine venöse Blutleitung 7 zu dem Patienten führt.

Die frische Dialysierflüssigkeit wird in einer Dialysierflüssigkeitsquelle 8 bereitgestellt. Von der Dialysierflüssigkeitsquelle 8 führt eine Dialysierflüssigkeitszuführleitung 9 zu einem Einlass der Dialysierflüssigkeitskammer 4 des Dialysators 1, während eine Dialysierflüssigkeitsabführleitung 30 von einem Auslass der Dialysierflüssigkeitskammer 4 zu einem Abfluss 11 führt. In der Dialysierflüssigkeitsabführleitung 30 ist eine Dialysierflüssigkeitspumpe 12 angeordnet.

Die Blutpumpe 6 ist eine peristaltische Schlauchpumpe, insbesondere eine Rollenpumpe, wobei die arterielle und venöse Blutleitung 5, 7 flexible Schlauchleitungen eines zur einmaligen Verwendung bestimmten Schlauchsets 20 (Disposable) sind.

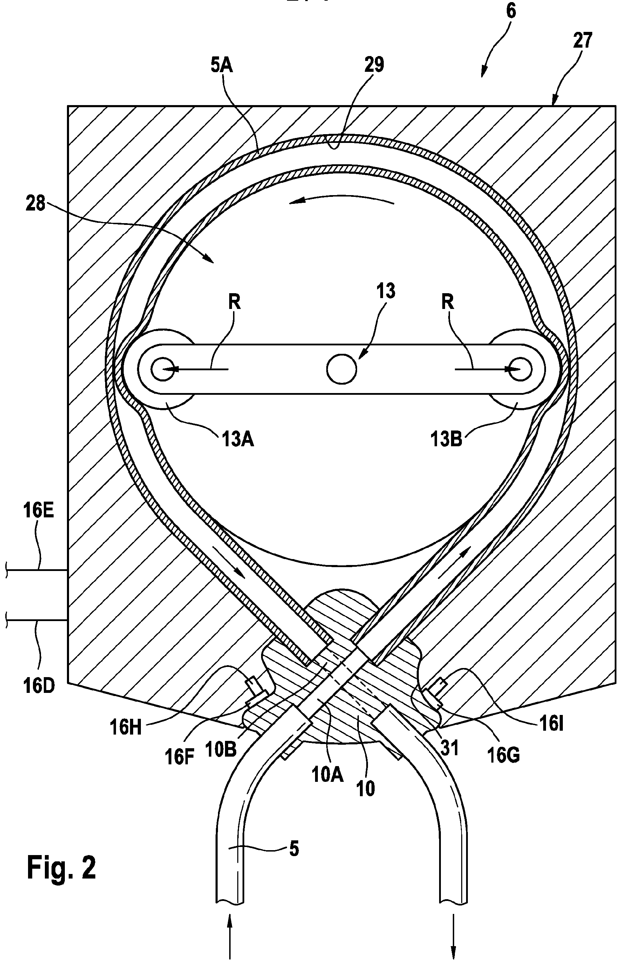

Fig. 2 zeigt die peristaltische Schlauchpumpe 6 in vereinfachter schematischer Darstellung. Die Schlauchpumpe 6 weist einen Gehäusekörper 27 mit einer Aufnahmeeinheit 28 auf, in die ein Schlauchsegment 5A der arteriellen Schlauchleitung 5 eingelegt ist. Das Schlauchsegment bildet eine Schlaufe, die passend in einem bogenförmigen Pumpenbett 29 liegt, das in der Aufnahmeeinheit 28 ausgebildet ist. Die sich kreuzenden Abschnitte der Schlauchleitung 5 sind mit einem Anschlussstück 10 fixiert, das in eine Ausnehmung 31 des Gehäusekörpers 27 passend, vorzugsweise einrastend, eingesetzt ist. Eine derartige Schlauchpumpe ist aus der WO 2005/111424 A1 bekannt.

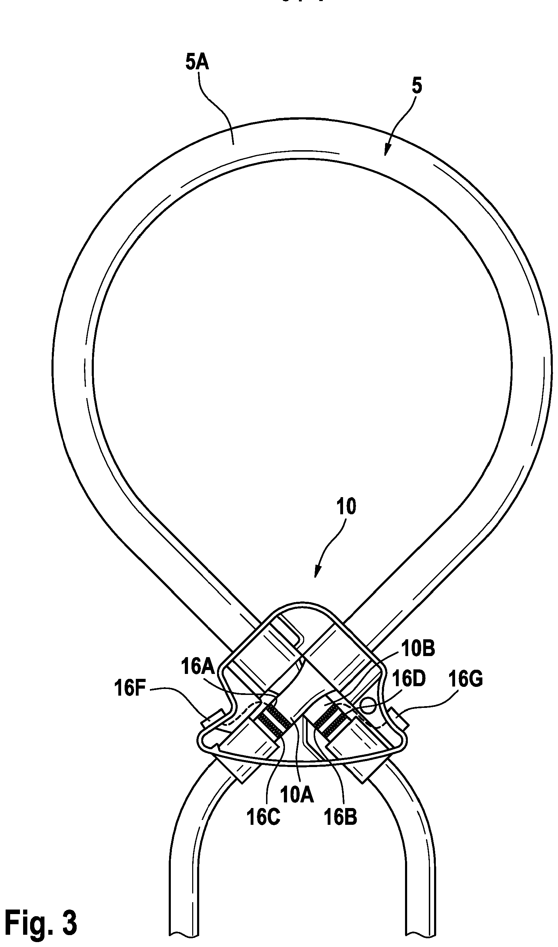

Die Schlauchpumpe weist bei dem vorliegenden Ausführungsbeispiel als Verdrängerkörper Rollen 13A, 13B auf, die an einem Rotor 13 drehbar gelagert sind, der von dem Pumpenbett 29 umschlossen wird. An dem Rotor 13 sind die Rollen in radialer Richtung R gegen die Schlauchleitung 5 federnd vorgespannt. Während des ordnungsgemäßen Betriebs quetschen die Rollen 13A, 13B die Schlauchleitung 5 vollständig ab, wie in Fig. 3 dargestellt ist. Da die Rollen 13A, 13B an dem Rotor 13 federnd vorgespannt sind, können die Rollen von der Schlauchleitung 5 abheben, wenn der Druck infolge einer Blockade der Flüssigkeitsströmung in der Schlauchleitung 5 stromab der Pumpe 6 ansteigt. Die Rollenpumpe kann auch mehr als zwei Rollen aufweisen.

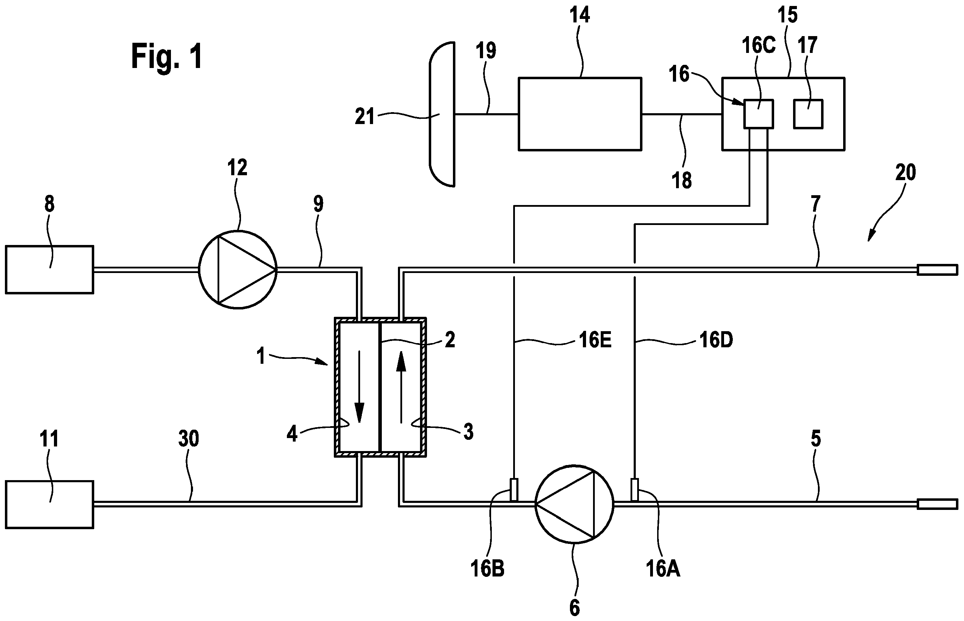

Die Blutbehandlungsvorrichtung weist eine zentrale Steuer- und Recheneinheit 14 auf und kann über weitere Komponenten verfügen, beispielsweise eine Bilanziereinrichtung oder Ultrafiltrationseinrichtung sowie verschiedene Sensoren zur Überwachung der Blutbehandlung, die in Fig. 1 aber nicht dargestellt sind.

Darüber hinaus verfügt die Blutbehandlungsvorrichtung über eine Überwachungsvorrichtung 15 zur Überwachung der Okklusion der Verdrängerkörper 13A, 13B der Schlauchpumpe. Die Überwachungsvorrichtung 15 weist eine Einrichtung 16 zum Messen des elektrischen Widerstandes oder einer mit dem elektrischen Widerstand korrelierenden Größe und eine Rechen- und Auswerteinheit 17 auf, die auch Bestandteil der zentralen Steuer- und Recheneinheit 14 der Blutbehandlungsvorrichtung sein können.

Die Einrichtung 16 zum Messen des elektrischen Widerstandes weist eine erste und eine zweite Elektrode 16A, 16B auf, zwischen denen der elektrische Widerstand mit einem Widerstands- oder Leitfähigkeitsmesser 16C gemessen wird. Die erste Elektrode 16A ist über eine erste elektrische Verbindungsleitung 16D und die zweite Elektrode 16B ist über eine zweite elektrische Verbindungsleitung 16E mit dem Widerstands- oder Leitfähigkeitsmesser 16C verbunden. Die beiden Elektroden 16A, 16B sind integraler Bestandteil des Anschlussstücks 10 der Schlauchleitung 5.

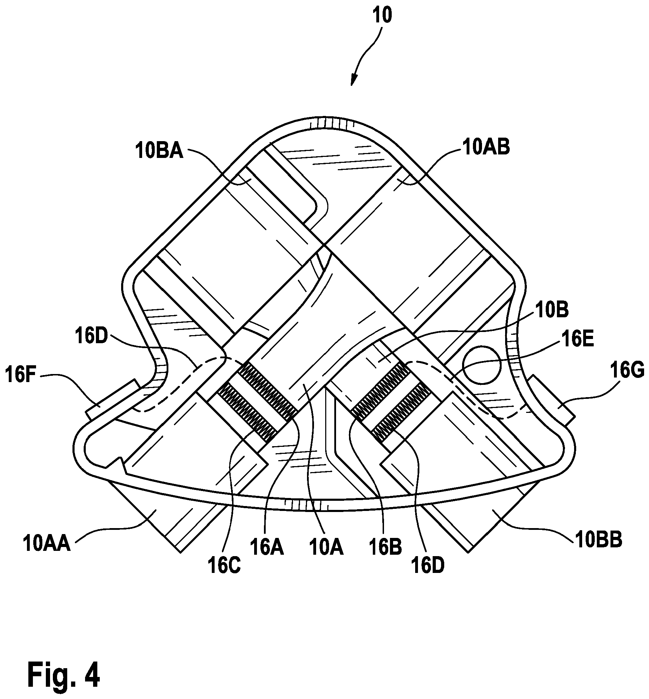

Die Figuren 3 und 4 zeigen ein Ausführungsbeispiel des in Fig. 1 schematisch dargestellten Anschlussstücks 10, in das die Elektroden 16A, 16B integriert sind. Fig. 4 zeigt das Anschlussstück 10 in vergrößerter Darstellung. Das Anschlussstück 10 ist ein vorzugsweise einstückiges Kunststoffteil, das aber auch mehrteilig sein kann, beispielsweise aus einem Gehäuseunterteil und einem Gehäuseoberteil besteht.

In dem Kunststoffteil sind ein erster Kanal 10A und ein zweiter Kanal 10B ausgebildet, die sich kreuzen. Die Endstücke des ersten Kanals 10A sind als Einlass- und Auslassstück 10AA, 10AB und die Endstücke des zweiten Kanals 10B sind als Einlass- und Auslassstück 10BA, 10BB ausgebildet. Die angrenzenden Schlauchleitungsabschnitte sind an das Einlass- und Auslassstück 10AA, 10AB und 10BA, 10BB des ersten und zweiten Kanals 10A, 10B angeschlossen, so dass das Blut des Patienten durch den einen Kanal 10A zu der Schlauchpumpe 6 und den anderen Kanal 10B von der Pumpe strömt.

Die erste Elektrode 16A ist an dem ersten Kanal 10A und die zweite Elektrode 16B ist an dem zweiten Kanal 10B vorgesehen. Die Elektroden 16A, 16B sind vorzugsweise ringförmige Elektroden, die in der Wandung des Kanals ausgebildet sind, so dass ein elektrischer Kontakt zwischen Elektrode und Blut hergestellt ist. Die ringförmigen Elektroden erstrecken sich vorzugsweise über den gesamten Querschnitt des Kanals.

Das Kunststoffteil 10 besteht aus einer ersten Komponente aus einem nicht leitfähigen Kunststoff und aus einer zweiten Komponente aus einem leitfähigen Kunststoff, wobei die ringförmigen Elektroden 16A, 16B von dem leitfähigen Kunststoff gebildet werden. Der leitfähige Kunststoff ist in den Figuren 3 und 4 mit einer schwarzen Schattierung dargestellt. Das Anschlussstück 10 wird im Zweikomponenten-Spritzgußverfahren (2K-Spritzgußverfahren) hergestellt, wobei in dem Bereich des Kanals 10A, 10B, in dem die Elektrode 16A, 16B ausgebildet werden soll, ein leitfähiger hämokompatibler Kunststoff verwendet wird. Die ringförmigen Elektroden sind vorzugsweise als ringförmige Fortsetzung der Schlauchleitung geformt. Der leitfähige Kunststoff kann beispielsweise ein Polymerverbund aus Polypropylen und Edelstahlfasern sein.

In das Anschlussstück 10 können noch weitere Elektroden 16C und 16D integriert werden, beispielsweise Elektroden für Ableitströme (Erdung) oder zur Ein- bzw. Auskopplung weiterer Signale.

In dem Anschlussstück 10 sind auch Abschnitte der elektrischen Anschlussleitungen 16D, 16E für die Elektroden 16A, 16B als Bahnen aus einem leitfähigen Kunststoff ausgebildet. Die Enden dieser leitfähigen Bahnen sind als Anschlusskontakte 16F, 16G ausgebildet sein, die beim Einsetzen des Anschlussstücks 10 in die Ausnehmung 31 der Aufnahmeeinheit 28 des Gehäusekörpers 27 mit entsprechenden Anschlusskontakten 16H, 16I an der Aufnahmeeinheit 28 kontaktieren. Dadurch kann die elektrische Verbindung zu dem Widerstands- oder Leitfähigkeitsmesser 16C besonders einfach hergestellt werden, wodurch die Handhabung weiter vereinfacht wird.

Die Rechen- und Auswerteinheit 17 kann unterschiedlich ausgebildet sein. Beispielsweise kann die Rechen- und Auswerteinheit 17 ein Mikrocomputer sein, auf dem ein Datenverarbeitungsprogramm (Software) läuft. Die Rechen- und Auswerteinheit 17 ist derart konfiguriert, dass der zwischen den Elektroden 16A, 16B gemessene Widerstand mit einem vorgegebenen Grenzwert verglichen wird. Die Messung erfolgt hochohmig. Solange die Verdrängerkörper 13A, 13B von der Schlauchleitung 5 nicht abgehoben sind, ist der gemessene Widerstand hoch. Wenn der Widerstand unter einen Grenzwert abfällt, wird darauf geschlossen, dass die Verdrängerkörper 13A, 13B von der Schlauchleitung 5 abgehoben sind. Anstelle des Widerstands kann auch die Leitfähigkeit gemessen werden. Solange die Verdrängerkörper 13A, 13B von der Schlauchleitung 5 nicht abgehoben sind, ist die gemessene Leitfähigkeit gering. Wenn die Leitfähigkeit über den Grenzwert ansteigt, wird darauf geschlossen, dass sich die Verdrängerkörper von der Schlauchleitung abgehoben haben. Die Größe des Widerstandes bzw. der Leitfähigkeit ist auch ein Maß für die Wegstrecke, um die sich die Verdrängerkörper 13A, 13B abgehoben haben, da der Querschnitt für den Durchtritt von leitfähiger Flüssigkeit, insbesondere Blut, beim Zurückschieben der Verdrängerkörper 13A, 13B entgegen der Federkraft zunimmt. Folglich kann durch Auswertung des Widerstandes bzw. der Leitfähigkeit auch auf die Position der Verdrängerkörper geschlossen werden, wobei die Abhängigkeit der Position der Verdrängerkörper von dem Widerstand bzw. der Leitfähigkeit aus den betreffenden Strömungsquerschnitten unter Berücksichtigung des Widerstandes bzw. der Leitfähigkeit des Fluids berechnet oder empirisch ermittelt werden kann. Eine entsprechende Funktion oder die entsprechenden Werte können in einem Speicher der Rechen- und Auswerteinheit 17 gespeichert sein, so dass die Rechen- und Auswerteinheit 17 die Position der Verdrängerkörper berechnen kann.

Die zentrale Steuer- und Recheneinheit 14 ist über eine Datenleitung 18 mit der Überwachungsvorrichtung 15 verbunden. Wenn die Überwachungsvorrichtung 15 ein Abheben der Verdrängerkörper 13A, 13B um einen bestimmten Betrag infolge eines Druckanstiegs in der Schlauchleitung erkennt, erzeugt die Überwachungsvorrichtung 15 ein Steuer- oder Alarmsignal, das die zentrale Rechen- und Steuereinheit 14 empfängt. Die zentrale Rechen- und Steuereinheit 14 ist über eine Datenleitung 19 mit einer Alarmeinheit 21 verbunden, die einen Alarm gibt. Die zentrale Rechen- und Steuereinheit 14 kann auch einen Eingriff in die Maschinensteuerung vornehmen, beispielsweise die Schlauchpumpe anhalten.

Die Überwachungsvorrichtung 15 kann auch ein Steuersignal (Datensignal) erzeugen, das von der Auslenkung der Verdrängerkörper 13A, 13B abhängig ist. Dieses Signal kann eine nicht dargestellte Anzeigeeinheit empfangen, auf der die Position der Verdrängerkörper angezeigt wird.

## Patentansprüche

1. Medizinische Behandlungsvorrichtung mit
einem Schlauchset (20), das eine oder mehrere Schlauchleitungen (5) aufweist, einer peristaltischen Schlauchpumpe (6) zum Fördern von Flüssigkeit, die eine Aufnahmeeinheit (28) mit einem Pumpenbett (29) zum Einlegen eines Schlauchsegments (5A) der Schlauchleitung (5) und bewegliche Okklusionskörper (13A, 13B) zur Beaufschlagung des in das Pumpenbett eingelegten Schlauchsegments aufweist, und
einer Überwachungsvorrichtung (15) zur Überwachung der Okklusion durch die Okklusionskörper (13A, 13B) der Schlauchpumpe (6),
wobei die Überwachungsvorrichtung (15) aufweist:
eine Einrichtung (16) zum Messen des elektrischen Widerstandes oder einer mit dem elektrischen Widerstand korrelierenden Größe zwischen einer ersten und einer zweiten Elektrode (16A, 16B), wobei die erste Elektrode (16A) stromauf der Okklusionskörper (13A, 13B) und die zweite Elektrode (16B) stromab der Okklusionskörper (16) der Schlauchpumpe (6) derart an der Schlauchleitung (5) angeordnet sind, dass ein elektrischer Kontakt zwischen der ersten und zweiten Elektrode (16A, 16B) und der in der Schlauchleitung (5) strömenden Flüssigkeit herstellbar ist, und
eine den elektrischen Widerstand oder die mit dem elektrischen Widerstand korrelierende Größe erfassende Rechen- und. Auswerteeinheit (17),
wobei die Aufnahmeeinheit (28) der Schlauchpumpe (6) ein bogenförmiges Pumpenbett (29) aufweist und das in die Aufnahmeeinheit einzulegende Schlauchsegment (5A) eine Schlaufe bildet, **dadurch gekennzeichnet, dass**
das Schlauchset (20) ein Anschlussstück (10) aufweist, mit dem die sich kreuzenden Abschnitte des eine Schlaufe bildenden Schlauchsegments (5A) fixiert sind,
wobei die erste und zweite Elektrode (16A, 16B) Bestandteil des Anschlussstücks (10) sind.

2. Medizinische Behandlungsvorrichtung nach Anspruch 1, **dadurch gekennzeichnet, dass** die Rechen- und Auswerteeinheit (17) derart konfiguriert ist, dass eine Veränderung des elektrischen Widerstandes erfasst wird, wobei auf eine mangelnde Okklusion der Okklusionskörper (13A, 13B) geschlossen wird, wenn der elektrische Widerstand einen vorgegebenen Grenzwert unterschreitet, oder
dass eine Veränderung der elektrischen Leitfähigkeit erfasst wird, wobei auf eine mangelnde Okklusion der Okklusionskörper (13A, 13B) geschlossen wird, wenn die elektrische Leitfähigkeit einen vorgegebenen Grenzwert überschreitet.

3. Medizinische Behandlungsvorrichtung nach Anspruch 1 oder 2, **dadurch gekennzeichnet, dass** die erste Elektrode (16A) und die zweite Elektrode (16B) in dem Bereich der sich kreuzenden Abschnitte des eine Schlaufe bildenden Schlauchsegments (5A) angeordnet sind.

4. Medizinische Behandlungsvorrichtung nach einem der Ansprüche 1 bis 3, **dadurch gekennzeichnet, dass** das Anschlussstück (10) ein Kunststoffteil ist, in dem ein erster Kanal (10A) mit einem Einlass (10AA) und einem Auslass (10AB) und ein zweiter Kanal (10B) mit einem Einlass (10BA) und einem Auslass (10BB) ausgebildet sind, und dass die Elektroden (16A, 16B) in der Wandung des ersten und zweiten Kanals (10A, 10B) ausgebildet sind.

5. Medizinische Behandlungsvorrichtung nach Anspruch 4, **dadurch gekennzeichnet, dass** der Kunststoff des Anschlussstücks (10) aus einer leitfähigen und einer nicht-leitfähigen Komponente besteht, wobei in dem Bereich des ersten und zweiten Kanals (10A, 10B), in dem die erste und zweite Elektrode (16A, 16B) ausgebildet sind, zur Ausbildung der Elektroden der Kunststoff ein leitfähiger Kunststoff ist.

6. Medizinische Behandlungsvorrichtung nach einem der Ansprüche 1 bis 5, **dadurch gekennzeichnet, dass** die erste und/oder zweite Elektrode (16A, 16B) ringförmige Elektroden sind.

7. Medizinische Behandlungsvorrichtung nach einem der Ansprüche 1 bis 6, **dadurch gekennzeichnet, dass** die medizinische Behandlungsvorrichtung eine Blutbehandlungsvorrichtung mit einem extrakorporalen Blutkreislauf ist, wobei das Schlauchset (20) ein Blutschlauchset mit einer Blutleitung (5) ist, die in die Aufnahmeeinheit (28) der Schlauchpumpe (6) einzulegen ist.

8. Schlauchset für eine medizinische Behandlungsvorrichtung mit einer oder mehreren Schlauchleitungen (5), wobei mindestens eine Elektrode (16A, 16B) zum Anschluss mindestens einer elektrischen Leitung (16D, 16E) einer Überwachungsvorrichtung (15) vorgesehen ist und die mindestens eine Elektrode (16A, 16B) Bestandteil eines aus einer leitfähigen und einer nicht-leitfähigen Komponente bestehenden Kunststoffteils (10) ist, in dem mindestens ein Kanal (10A, 10B) ausgebildet ist, der einen Einlass (10AA) aufweist, an den ein Schlauchsegment der Schlauchleitung angeschlossen ist, und einen Auslass (10AB) aufweist, an den ein Schlauchsegment der Schlauchleitung angeschlossen ist, und wobei der Kunststoff in einem Bereich des Kunststoffteils, in dem Elektrode (16A, 16B) ausgebildet ist, ein leitfähiger Kunststoff ist,
wobei ein Schlauchsegment (5A) der Schlauchleitung (5) eine Schlaufe zum Einlegen in ein bogenförmiges Pumpenbett (29) einer Aufnahmeeinheit (28) einer peristaltischen Schlauchpumpe (6) bildet, wobei das Kunststoffteil ein Anschlussstück (10) ist, mit dem die sich kreuzenden Abschnitte des Schlauchsegments (5A) fixiert sind.

9. Schlauchset nach Anspruch 8, **dadurch gekennzeichnet, dass** eine erste und eine zweite Elektrode zum Anschluss einer ersten und einer zweiten elektrischen Leitung (16D, 16E) einer Überwachungsvorrichtung (15) zur Überwachung der Okklusion durch Okklusionskörper (13A, 13B) der Schlauchpumpe (6) vorgesehen ist.

10. Schlauchset nach Anspruch 9, **dadurch gekennzeichnet, dass** in dem Anschlussstück (10) ein erster Kanal (10A) mit einem Einlass (10AA) und einem Auslass (10AB) und ein zweiter Kanal (10B) mit einem Einlass (10BA) und einem Auslass (10BB) ausgebildet sind, und dass die Elektroden (16A, 16B) in der Wandung des ersten und zweiten Kanals (10A, 10B) ausgebildet sind.

11. Schlauchset nach Anspruch 9 oder 10, **dadurch gekennzeichnet, dass** die Elektroden (16A, 16B) ringförmige Elektroden sind.

## Claims

1. Medical treatment device comprising
a hose set (20) which comprises one or more hose lines (5),
a peristaltic pump (6) for conveying fluid, which comprises a receiving unit (28) having a pump bed (29) for inserting a hose segment (5A) of the hose line (5), and movable occlusion elements (13A, 13B) for acting on the hose segment inserted into the pump bed, and
a monitoring device (15) for monitoring the occlusion through the occlusion elements (13A, 13B) of the peristaltic pump (6),
the monitoring device (15) comprising:
a device (16) for measuring the electrical resistance or a variable which correlates with the electrical resistance between a first and a second electrode (16A, 16B), the first electrode (16A) being arranged on the hose line (5) upstream of the occlusion elements (13A, 13B) and the second electrode (16B) being arranged on the hose line downstream of the occlusion elements (16) of the peristaltic pump (6) such that an electrical contact can be produced between the first and second electrode (16A, 16B) and the fluid flowing in the hose line (5), and
a calculation and evaluation unit (17) which detects the electrical resistance or a variable which correlates with the electrical resistance,
the receiving unit (28) of the peristaltic pump (6) comprising an arcuate pump bed (29) and the hose segment (5A) to be inserted into the receiving unit forming a loop,
**characterised in that**
the hose set (20) comprises a connecting piece (10), by means of which the intersecting portions of the hose segment (5A) forming a loop are fixed, the first and second electrode (16A, 16B) being component parts of the connecting piece (10).

2. Medical treatment device according to claim 1, **characterised in that** the calculation and evaluation unit (17) is designed such that a change in the electrical resistance is detected, a lack of occlusion of the occlusion elements (13A, 13B) being concluded if the electrical resistance falls below a predetermined threshold value, or
such that a change in the electrical conductivity is detected, a lack of occlusion of the occlusion elements (13A, 13B) being concluded if the electrical conductivity exceeds a predetermined threshold value.

3. Medical treatment device according to claim 1 or 2, **characterised in that** the first electrode (16A) and the second electrode (16B) are arranged in the region of the intersecting portions of the hose segment (5A) that forms a loop.

4. Medical treatment device according to any of claims 1 to 3, **characterised in that** the connecting piece (10) is a plastics part in which a first channel (10A) comprising an inlet (10AA) and an outlet (10AB), and a second channel (10B) comprising an inlet (10BA) and an outlet (10BB) are formed, and **in that** the electrodes (16A, 16B) are formed in the wall of the first and second channels (10A, 10B).

5. Medical treatment device according to claim 4, **characterised in that** the plastics material of the connecting piece (10) consists of a conductive and a non-conductive component, the plastics material in the region of the first and second channel (10A, 10B) in which the first and second electrodes (16A, 16B) are formed being a conductive plastics material for forming the electrodes.

6. Medical treatment device according to any of claims 1 to 5, **characterised in that** the first and/or second electrodes (16A, 16B) are annular electrodes.

7. Medical treatment device according to any of claims 1 to 6, **characterised in that** the medical treatment device is a blood treatment device comprising an extracorporeal blood circuit, the hose set (20) being a blood hose set comprising a blood line (5) which is to be inserted into the receiving unit (28) of the peristaltic pump (6).

8. Hose set for a medical treatment device, comprising one or more hose lines (5), at least one electrode (16A, 16B) being provided for connecting at least one electrical line (16D, 16E) of a monitoring device (15), and the at least one electrode (16A, 16B) being a component part of a plastics part (10) consisting of a conductive and a non-conductive component, in which plastics part at least one channel (10A, 10B) is formed which comprises an inlet (10AA), to which a hose segment of the hose line is connected, and an outlet (10AB), to which a hose segment of the hose line is connected, the plastics material in a region of the plastics part in which the electrode (16A, 16B) is formed being a conductive plastics material,
a hose segment (5A) of the hose line (5) forming a loop for inserting into an arcuate pump bed (29) of a receiving unit (28) of a peristaltic pump (6), the plastics part being a connecting piece (10) by means of which the intersecting portions of the hose segments (5A) are fixed.

9. Hose set according to claim 8, **characterised in that** a first and a second electrode are provided for connecting a first and a second electrical line (16D, 16E) of a monitoring device (15) for monitoring the occlusion through occlusion elements (13A, 13B) of the peristaltic pump (6).

10. Hose set according to claim 9, **characterised in that** a first channel (10A) comprising an inlet (10AA) and an outlet (10AB), and a second channel (10B) comprising an inlet (10BA) and an outlet (10BB) are formed in the connecting piece (10), and **in that** the electrodes (16A, 16B) are formed in the wall of the first and second channels (10A, 10B).

11. Hose set according to claim 9 or 10, **characterised in that** the electrodes (16A, 16B) are annular electrodes.

## Revendications

1. Dispositif de traitement médical avec
un ensemble de tuyaux flexibles (20), qui présente un ou plusieurs conduits en tuyau flexible (5),
une pompe péristaltique à tuyau flexible (6) pour refouler du liquide, qui présente une unité de logement (28) avec un lit de pompe (29) pour placer un segment de tuyau flexible (5A) du conduit en tuyau flexible (5) et des corps d'occlusion (13A, 13B) mobiles pour soumettre à une action le segment de tuyau flexible placé dans le lit de pompe, et
un dispositif de surveillance (15) pour surveiller l'occlusion par les corps d'occlusion (13A, 13B) de la pompe à tuyau flexible (6),
dans lequel le dispositif de surveillance (15) présente :
un système (16) pour mesurer la résistance électrique ou une grandeur en corrélation avec la résistance électrique entre une première et une deuxième électrode (16A, 16B), dans lequel la première électrode (16A) est disposée en amont des corps d'occlusion (13A, 13B) et la deuxième électrode (16B) est disposée en aval des corps d'occlusion (16) de la pompe à tuyau flexible (6) de telle manière au niveau du conduit en tuyau flexible (5) qu'un contact électrique entre la première et la deuxième électrode (16A, 16B) et le liquide s'écoulant dans le conduit en tuyau flexible (5) peut être établi, et
une unité de calcul et d'évaluation (17) détectant la résistance électrique ou la grandeur en corrélation avec la résistance électrique,
dans lequel
l'unité de logement (28) de la pompe à tuyau flexible (6) présente un lit de pompe (29) en forme d'arc, et
le segment de tuyau flexible (5A) à placer dans l'unité de logement forme une boucle, **caractérisé en ce que**
l'ensemble de tuyaux flexibles (20) présente une pièce de raccordement (10), par laquelle les sections se croisant du segment de tuyau flexible (5A) formant une boucle sont bloquées,
dans lequel la première et la deuxième électrode (16A, 16B) font partie intégrante de la pièce de raccordement (10).

2. Dispositif de traitement médical selon la revendication 1, **caractérisé en ce que** l'unité de calcul et d'analyse (17) est configurée de telle manière qu'une modification de la résistance électrique est détectée, dans lequel une absence d'occlusion des corps d'occlusion (13A, 13B) est déduite quand la résistance électrique est inférieure à une valeur de limite spécifiée, ou
qu'une modification de la conductivité électrique est détectée, dans lequel une absence d'occlusion des corps d'occlusion (13A, 13B) est déduite quand la conductivité électrique dépasse une valeur de limite spécifiée.

3. Dispositif de traitement médical selon la revendication 1 ou 2, **caractérisé en ce que** la première électrode (16A) et la deuxième électrode (16B) sont disposées dans la zone des sections se croisant du segment de tuyau flexible (5A) formant une boucle.

4. Dispositif de traitement médical selon l'une quelconque des revendications 1 à 3, **caractérisé en ce que** la pièce de raccordement (10) est une partie en matière plastique, dans laquelle sont réalisés un premier canal (10A) avec une entrée (10AA) et une sortie (10AB) et un deuxième canal (10B) avec une entrée (10BA) et une sortie (10BB), et que les électrodes (16A, 16B) sont réalisées dans la paroi du premier et du deuxième canal (10A, 10B).

5. Dispositif de traitement médical selon la revendication 4, **caractérisé en ce que** la matière plastique de la pièce de raccordement (10) est constituée d'un composant conducteur et d'un composant non conducteur, dans lequel une matière plastique conductrice est dans la zone du premier et du deuxième canal (10A, 10B), dans laquelle la première et la deuxième électrode (16A, 16B) sont réalisées, pour réaliser les électrodes.

6. Dispositif de traitement médical selon l'une quelconque des revendications 1 à 5, **caractérisé en ce que** la première et/ou la deuxième électrode (16A, 16B) sont des électrodes de forme annulaire.

7. Dispositif de traitement médical selon l'une quelconque des revendications 1 à 6, **caractérisé en ce que** le dispositif de traitement médical est un dispositif de traitement du sang avec un circuit de sang extracorporel, dans lequel l'ensemble de tuyaux flexibles (20) est un ensemble de tuyaux flexibles à sang avec un conduit de sang (5) qui est à placer dans l'unité de logement (28) de la pompe à tuyau flexible (6).

8. Ensemble de tuyaux flexibles pour un dispositif de traitement médical avec un ou plusieurs conduits à tuyau flexible (5), dans lequel au moins une électrode (16A, 16B) est prévue pour raccorder au moins un conduit électrique (16D, 16E) d'un dispositif de surveillance (15), et l'au moins une électrode (16A, 16B) fait partie intégrante d'une partie en matière plastique (10) constituée d'un composant conducteur et d'un composant non conducteur, dans laquelle au moins un canal (10A, 10B) est réalisé, qui présente une entrée (10AA), à laquelle un segment de tuyau flexible du conduit en tuyau flexible est raccordé, et présente une sortie (10AB), à laquelle un segment de tuyau flexible du conduit en tuyau flexible est raccordé, et dans lequel la matière plastique dans une zone de la partie en matière plastique, dans laquelle une électrode (16A, 16B) est réalisée, est une matière plastique conductrice,
dans lequel un segment de tuyau flexible (5A) du conduit en tuyau flexible (5) forme une boucle destinée à être placée dans un lit de pompe (29) en forme d'arc d'une unité de logement (28) d'une pompe péristaltique à tuyau flexible (6), dans lequel la partie en matière plastique est une pièce de raccordement (10), par laquelle les sections se croisant du segment de tuyau flexible (5A) sont bloquées.

9. Ensemble de tuyaux flexibles selon la revendication 8, **caractérisé en ce qu'**une première et une deuxième électrode sont prévues pour raccorder un premier et un deuxième conduit (16D, 16E) électrique d'un dispositif de surveillance (15) pour surveiller l'occlusion par des corps d'occlusion (13A, 13B) de la pompe à tuyau flexible (6).

10. Ensemble de tuyaux flexibles selon la revendication 9, **caractérisé en ce que** sont réalisés dans la pièce de raccordement (10) un premier canal (10A) avec une entrée (10AA) et une sortie (10AB) et un deuxième canal (10B) avec une entrée (10BA) et une sortie (10BB), et que les électrodes (16A, 16B) sont réalisées dans la paroi du premier et du deuxième canal (10A, 10B).

11. Ensemble de tuyaux flexibles selon la revendication 9 ou 10, **caractérisé en ce que** les électrodes (16A, 16B) sont des électrodes annulaires.
